# FASCICULE DE BREVET EUROPEEN

(11) **EP 0 719 568 B1**
(45) Date de publication et mention de la délivrance du brevet: **27.12.2006**
(21) Numéro de dépôt: 95402976.5
(22) Date de dépôt: 29.12.1995
(51) Int. Cl.: A61N 1/365, A61N 1/37

(54) **Dispositif médical implantable actif avec des moyens de distinction entre les états de repos et non-repos du patient**
Implantierbare aktive medizinische Vorrichtung mit Mittel zur Unterscheidung zwischen Ruhe- bzw. Aktivzustand des Patienten
Active implantable medical device with means for distinguishing between rest and non-rest states of the patient

(30) Priorité: 30.12.1994 FR 9415912
(43) Date de publication de la demande: 03.07.1996
(73) Titulaire: ELA MEDICAL (Société anonyme), 92541 Montrouge (FR)
(72) Inventeur: Bonnet, Jean-Luc, F-92170 Vanves (FR)
(74) Mandataire: Dupuis-Latour, Dominique

(56) Documents cités:
- EP-A- 0 317 065
- WO-A-93/08873
- WO-A-93/20889

## Description

La présente invention concerne les "dispositifs médicaux implantables actifs" tels que définis par la directive 90/385/CEE du 20 juin 1990 du Conseil des communautés européennes., notamment les stimulateurs cardiaques ou défibrillateurs, dont le fonctionnement est asservi à un paramètre recueilli à l'aide d'un capteur.

À cet égard, bien que dans la suite de la description on fasse principalement référence au cas d'un stimulateur cardiaque, l'invention est applicable de façon générale à une très grande variété de dispositifs électroniques.

De tels appareils sont connus pour adapter leurs actions, par exemple la fréquence de stimulation dans le cas d'un stimulateur cardiaque (comme décrit par le WO-A-93 20889) ou la détermination du moment où une thérapie de choc devra être appliquée dans le cas d'un défibrillateur (comme décrit par le EP-A-0 317 065), à la valeur mesurée ou calculée d'un paramètre représentatif des besoins métaboliques du porteur de l'appareil.

Le EP-A-0 089 014 décrit l'utilisation de la mesure de la fréquence respiratoire pour faire varier la fréquence instantanée de la stimulation cardiaque. De multiples paramètres, tels que la ventilation-minute, la mesure de la saturation d'oxygène dans le sang, la température ou l'accélération ont été utilisés comme paramètres d'asservissement.

Dans les cas des stimulateurs cardiaques, tous ces systèmes concourent à augmenter la fréquence des impulsions de stimulation lorsque l'on détecte une activité croissante du patient porteur de l'appareil, et à diminuer cette fréquence jusqu'à une valeur plancher en cas de diminution de cette activité, tout particulièrement durant les phases de repos du patient.

Le EP-A-0 493 222 décrit un procédé de corrélation entre, d'une part, les deux valeurs extrêmes Fc_{bas} et Fcₘₐₓ de l'excursion de la fréquence de stimulation et, d'autre part, les valeurs X_{bas} et Xₘₐₓ, respectivement valeur de repos et valeur d'activité maximale, calculées à partir des informations recueillies par le capteur d'asservissement. Ce procédé de corrélation est connu sous le nom de "calibration automatique de l'asservissement", et le document expose un procédé pour déterminer la valeur de X_{bas} dans le cas de l'utilisation de la ventilation-minute comme paramètre d'asservissement, cette valeur de la ventilation-minute de repos étant alors appelée "VErepos".

Cette dernière valeur est obtenue par le calcul d'une valeur moyenne sur un intervalle de l'ordre de 24 heures, incluant donc aussi bien des périodes d'activité que des périodes de sommeil du porteur de l'appareil.

Les auteurs de la présente invention ont toutefois constaté que, durant les phases de sommeil, les valeurs de VErepos peuvent être de plus de 50 % inférieures aux valeurs de ce même paramètre enregistré pendant les périodes de veille.

Dans le document précité, de telles variations importantes ne peuvent pas être prises en considération. Or cette valeur de VErepos est utilisée pour la calibration automatique de l'asservissement de l'appareil lors de la fixation du point de fonctionnement en relation avec la fréquence minimale de stimulation Fc_{bas}. Une fausse estimation de cette valeur VErepos peut donc conduire à un mauvais réglage de la fréquence de stimulation par rapport aux besoins réels du porteur de l'appareil.

L'un des buts de la présente invention est de proposer un dispositif médical implantable actif, notamment un stimulateur cardiaque asservi à un paramètre par l'intermédiaire d'au moins un capteur, permettant de surveiller de façon continue le niveau d'activité de ce capteur afin d'établir une valeur de repos apte à permettre une calibration correcte de cet asservissement.

Un autre but de la présente invention est de proposer un tel dispositif permettant d'opérer une distinction entre les phases de repos (nocturne ou diurne) du porteur de l'appareil et d'autres phases d'activité. Jusqu'à présent, on ne savait en effet effectuer une telle distinction que de façon approximative et arbitraire, à l'aide d'une horloge interne commutant à heures fixes certains réglages du dispositif, comme cela est par exemple enseigné dans le US-A-5143 065.

Le dispositif de l'invention permettra ainsi de déterminer un critère, qui sera appelé par la suite "critère d'activité du capteur", correspondant aux phases de repos ou bien d'activité du porteur de l'appareil.

À cet effet, l'invention propose un dispositif tel qu'énoncé dans la revendication 1.

Un certain nombre de caractéristiques subsidiaires avantageuses sont énoncées dans les sous-revendications.

D'autres caractéristiques et avantages de l'invention apparaîtront à la lecture de la description ci-dessous, faite en référence aux dessins annexés.
La figure 1 est un organigramme de la phase d'initialisation mise en oeuvre par l'invention, lors de la mise en route ou de la réinitialisation volontaire opérée par un praticien.
La figure 2 est un organigramme de la phase normale de fonctionnement, au cours de laquelle on détermine en continu différentes variables selon l'invention.
La figure 3 est un organigramme de l'étape de mise à jour de la variable ValeurSuiviCapteur.
La figure 4 est un organigramme de l'étape de mise à jour de la variable ValeurReposCapteur.
La figure 5 est un organigramme de l'étape d'incrémentation de la variable MoyenneCapteur24h.
La figure 6 est un organigramme de l'étape de mise à jour de la variable MoyenneCapteur24h.
La figure 7 est un organigramme de l'étape de détermination de la variable ÉtatCapteur dans le cas de l'utilisation d'un paramètre physiologique (ventilation-minute, température, etc.).
La figure 8 est une variante de la figure 7, dans le cas de l'emploi d'un paramètre physique tel que l'accélération.
La figure 9 est un exemple montrant l'évolution dans le temps des différentes variables mises en oeuvre par l'invention, enregistrées sur un intervalle de temps de 24 heures.

Pour des raisons de clarté de la description, on fera référence à un capteur d'un paramètre physiologique qui est la "ventilation-minute". Mais l'invention est bien entendue applicable semblablement à d'autres paramètres physiologiques tels que ceux qui ont été indiqués dans l'introduction de la présente description, tout autre recueil ou mesure d'un paramètre physiologique, apte à être utilisé pour des fonctions telles qu'un asservissement de dispositif implantable actif (ou autres fonctions), peut être substitué à la ventilation-minute sans sortir du cadre de la présente invention.

Le principe de l'invention peut s'appliquer également à l'asservissement d'un dispositif implantable actif par un paramètre non physiologique tel que l'accélération mesurée par un capteur interne au boîtier, comme cela est notamment décrit dans le US-A-5 330 510.

La mesure de la ventilation-minute est en elle-même bien connue ; elle est décrite notamment dans le document *Breath-by-Breath Minute Ventilation Measurement Can Provide a Fast Response,* par J. L. Bonnet, L. Krämer, M. Limousin, EUR J. C. P. E., 1994, Vol. 4, Abstract n° 329, et elle est mise en oeuvre dans les appareils *Chorus RM 7034* fabriqués par la société ELA Médical.

Par ailleurs, le dispositif décrit s'appuie de façon préférentielle sur une architecture composée d'un microprocesseur associé à des instructions de programmation en mémoire morte ROM et de circuits logiques et analogiques en eux-mêmes connus. Une telle structure est par exemple celle employée dans les stimulateurs cardiaques *Chorus* fabriqués par la société ELA Médical. Bien qu'elle ne présente pas les avantages de souplesse de mise en oeuvre de la solution à microprocesseur, une conception en circuits logiques dédiés et câblés est toutefois parfaitement envisageable et entre également dans le cadre de l'invention.

On va maintenant décrire les diverses modalités de mise en oeuvre de l'invention.

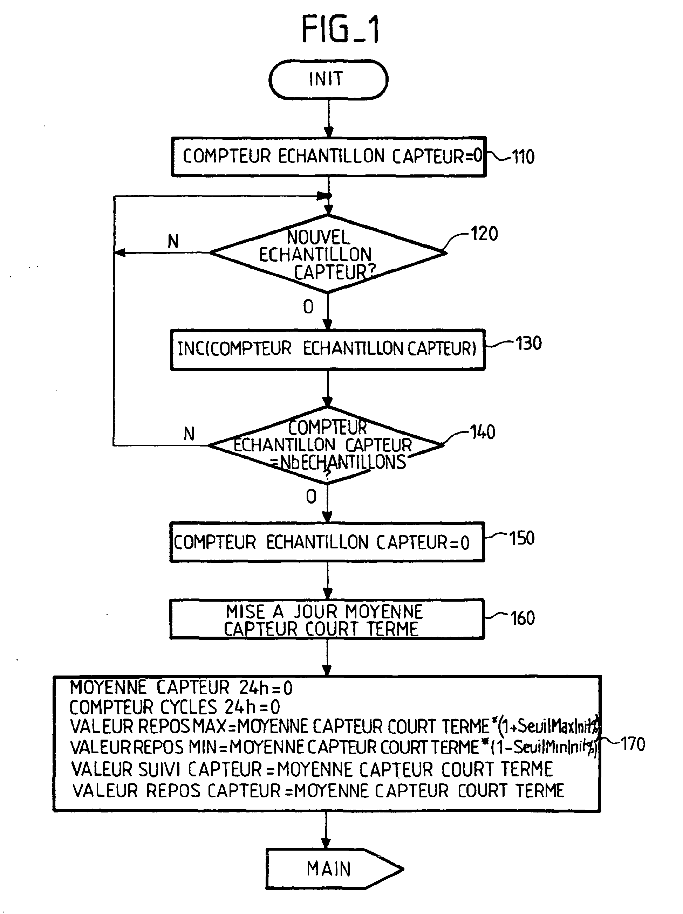

On va tout d'abord décrire le déroulement de la phase d'initialisation, en référence à la figure 1.

En effet, le calcul de certaines variables (MoyenneCapteur24h, ValeurSuiviCapteur et ValeurReposCapteur, qui seront explicitées plus bas) peut s'effectuer selon deux modalités différentes, selon que le dispositif se trouve dans une phase d'initialisation ou bien en régime de fonctionnement permanent continu, régime que l'on appellera "phase normale de fonctionnement".

La phase d'initialisation est mise en oeuvre lors de la première mise en fonctionnement du dispositif médical, par exemple au moment de l'implantation de celui-ci ou sur une demande externe spécifique ; elle vise à doter le dispositif de valeurs initiales qui seront ensuite automatiquement et ultérieurement affinées au cours du temps dans la phase normale de fonctionnement.

Dans cette phase d'initialisation, le dispositif garde en mémoire les valeurs de ventilation correspondant, typiquement, à 32 échantillons de mesure de la ventilation-minute (étapes 110 à 140 sur la figure 1) un échantillon correspondant à la détermination de la ventilation-minute (VE) sur un cycle respiratoire.

Quand la valeur de CompteurÉchantillonCapteur atteint 32, le dispositif remet à zéro cette valeur (étape 150) et calcule la moyenne des 32 valeurs successivement acquises, que l'on appellera MoyenneCapteurCourtTerme (étape 160).

On initialise alors les différentes variables utilisées (étape 170) :
- on remet à zéro le compteur CompteurCycles24h ainsi que la variable MoyenneCapteur24h,
- on donne à ValeurSuiviCapteur et ValeurReposCapteur la valeur MoyenneCapteurCourtTerme que l'on vient de déterminer,
- on donne à ValeurReposMax la valeur MoyenneCapteurCourtTerme affectée d'un coefficient prédéterminé (1 + SeuilMaxInit %), valant typiquement 50 %, et
- on donne à ValeurReposMin la valeur MoyenneCapteurCourtTerme affectée d'un coefficient prédéterminé (1 - SeuilMinInit %), valant typiquement 50 %.

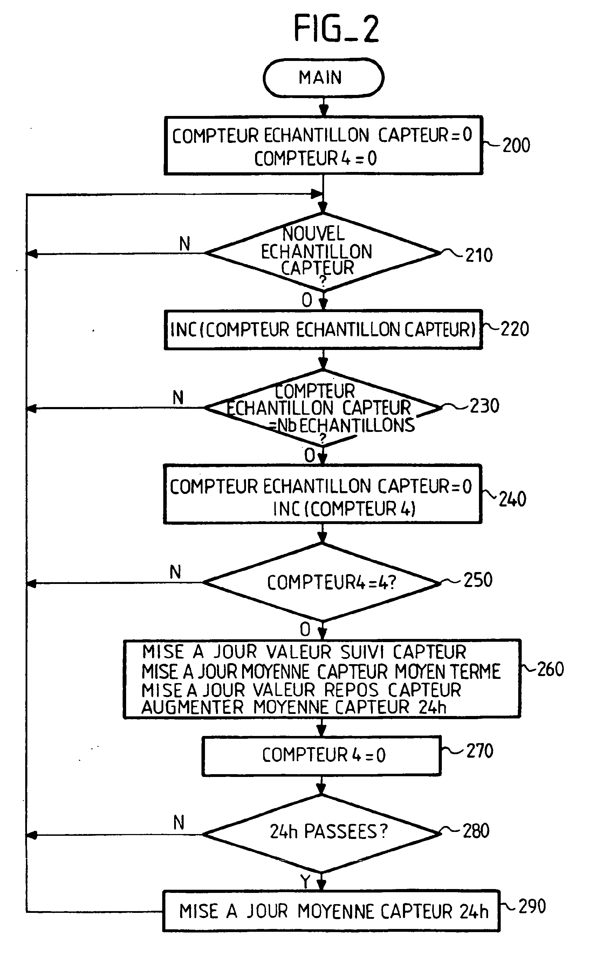

Ces diverses variables une fois initialisées servent de valeurs initiales dans la phase normale de fonctionnement, que l'on va maintenant décrire en référence aux figures 2 à 8.

Le déroulement général de cette phase est illustrée de façon générale sur la figure 2.

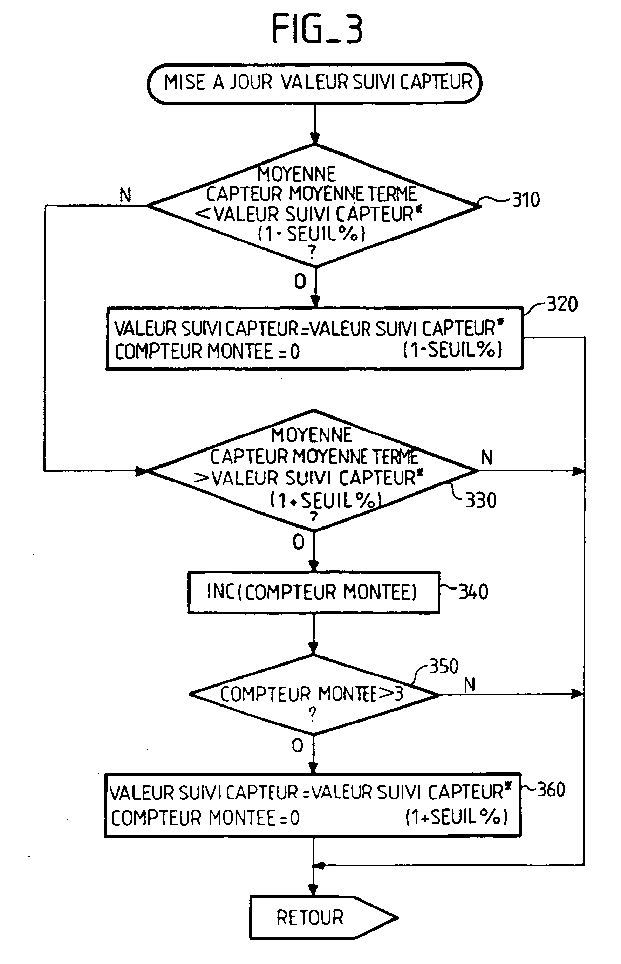

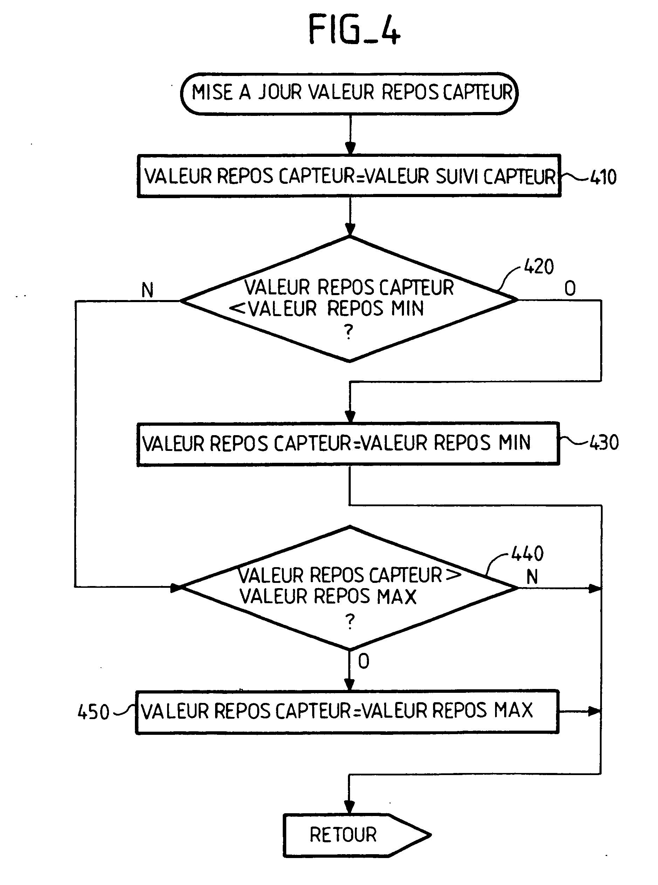

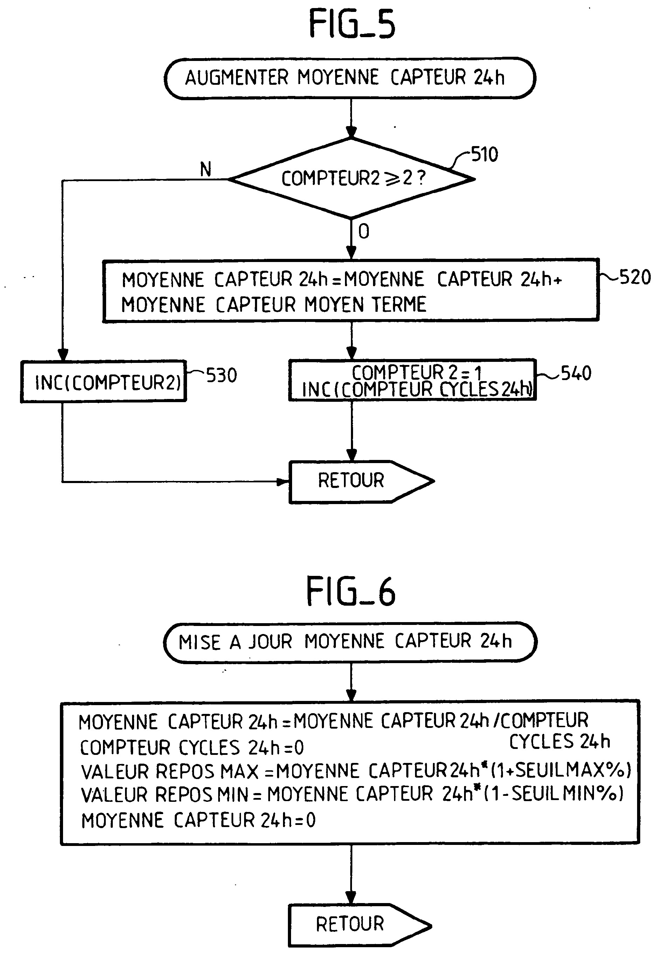

Le dispositif implantable exécute les étapes suivantes :
- remise à zéro des deux compteurs CompteurÉchantillonCapteur et Compteur4 (étape 200)
- recueil des échantillons successifs produits par le capteur (étapes 210 à 250).
- tous les 128 échantillons, c'est-à-dire après quatre répétitions du recueil de 32 échantillons (CompteurÉchantillonCapteur = 32 et Compteur4 = 4), le dispositif met à jour les variables (étape 260) :
   - ValeurSuiviCapteur, conformément à l'organigramme de la figure 3, que l'on décrira ci-dessous,
   - MoyenneCapteurMoyenTerme : cette valeur est la moyenne des 128 échantillons précédemment mesurés (bien entendu, les nombres de 128 et de 32 échantillons ci-dessus peuvent être remplacés par toute autre valeur quelconque, en fonction de la mémoire du dispositif et de sa puissance de calcul),
   - ValeurReposCapteur, conformément à l'organigramme de la figure 4, que l'on décrira ci-dessous, et
   - MoyenneCapteur24h, conformément à l'organigramme de la figure 5, que l'on décrira ci-dessous.

La figure 3 illustre la mise à jour périodique de la variable ValeurSuiviCapteur.

Cette variable sert à déterminer le niveau d'activité du capteur à l'issue de l'étape 260, c'est-à-dire après 128 cycles de mesure des échantillons. Elle est en outre utilisée pour le calcul des variables ValeurReposCapteur et MoyenneCapteur24h.

Elle est calculée de la manière suivante :
- si la valeur de MoyenneCapteurMoyenTerme est comprise dans les limites de ValeurSuiviCapteur ± Seuil % (Seuil étant une valeur prédéterminée, typiquement de 6,25 %), alors ValeurSuiviCapteur n'est pas modifiée (étapes 310 et 330),
- si la valeur de MoyenneCapteurMoyenTerme est devenue inférieure à ValeurSuiviCapteur - Seuil %, on considère que le niveau d'activité capté a diminué, et l'on diminue alors ValeurSuiviCapteur d'une quantité Seuil % et remet à zéro le compteur CompteurMontée (étapes 310 et 320), et
- si la valeur de MoyenneCapteurMoyenTerme est devenue supérieure à ValeurSuiviCapteur + Seuil %, alors on incrémente CompteurMontée d'une unité (étapes 310, 330 et 340). Si CompteurMontée atteint 4 (nombre choisi de façon arbitraire, mais correspondant à une situation typique), on considère que l'activité captée a augmenté et l'on augmente alors ValeurSuiviCapteur d'une quantité Seuil % et on remet à zéro CompteurMontée (étapes 350 et 360).

La figure 4 illustre la mise à jour périodique de la variable ValeurReposCapteur.

On donne par défaut à ValeurReposCapteur la valeur ValeurSuiviCapteur déterminée précédemment (étape 410). Mais ValeurReposCapteur est toutefois limitée à deux bornes dépendant de Moyenne-Capteur24h, telles que :
- si ValeurReposCapteur est inférieure à ValeurReposMin, alors on donne à ValeurReposCapteur la valeur ValeurReposMin (étapes 420 et 430),
- si ValeurReposCapteur est supérieure à ValeurReposMax, alors on donne à ValeurReposCapteur la valeur ValeurReposMax (étapes 420, 440 et 450), et

La détermination des valeurs ValeurReposMin et ValeurReposMax sera expliquée ci-après, en référence à la figure 6, notamment dans le cas où ces valeurs ne correspondent pas à celles établies au moment de la phase d'initialisation (étape 170).

Les figures 5 et 6 illustrent la détermination de la variable MoyenneCapteur24h.

Cette variable est d'abord incrémentée de la manière précisée sur l'organigramme de la figure 5, mis en oeuvre au cours de l'étape 260 de la figure 2.

Suivant la valeur de Compteur2 (compteur qui ne peut prendre que les valeurs 1 ou 2), on augmente la variable MoyenneCapteur24h de la valeur de MoyenneCapteurMoyenTerme (étape 520), et on incrémente un compteur CompteurCycles24h (étape 540).

À l'issue d'une période de 24 heures (étape 280 de la figure 2), calculée à partir d'une horloge interne du dispositif ou à partir du nombre d'itérations des phases précédentes correspondant approximative-ment à une durée de 24 heures, le dispositif met à jour la variable MoyenneCapteur24h (étape 290 de la figure 2).

Les différentes opérations aboutissant à cette mise à jour de MoyenneCapteur24h sont explicitées en 610 sur la figure 6.

Plus précisément, la variable MoyenneCapteur24h prend la valeur de la moyenne des sommations de MoyenneCapteurMoyenTerme établies à l'étape 520, moyenne qui est calculée en divisant le total des sommations par la valeur CompteurCycles24h déterminée à l'étape 540 décrite ci-dessus (figure 5). À l'étape 610 (figure 6), le dispositif fixe des valeurs ValeurReposMax et ValeurReposMin, calculées à partir du résultat précédent portant sur la valeur MoyenneCapteur24h :
- la valeur maximale, ValeurReposMax, de ValeurRepos est fixée à MoyenneCapteur24h x (1+SeuilMax%), avec typiquement une valeur SeuilMax% = 50 %, et
- la valeur minimale, ValeurReposMin, de ValeurRepos est fixée à MoyenneCapteur24h x (1-SeuilMin%), avec typiquement une valeur SeuilMin% = 0.

À l'issue de l'étape 610, MoyenneCapteur24h est initialisée à zéro, ainsi que CompteurCycles24h.

On notera que la détermination de la variable ValeurRepos, en combinaison avec les deux bornes de variation extrêmes ValeurReposMax et ValeurReposMin (elles-mêmes dépendantes de la variable MoyenneCapteur24h) permet d'établir de façon parfaitement appropriée le point bas de la calibration automatique de la courbe d'asservissement qui est décrite dans le EP-A-0 493 222 précité, où l'on pourra faire correspondre à ValeurRepos la fréquence de stimulation Fc_{bas} programmée par le praticien.

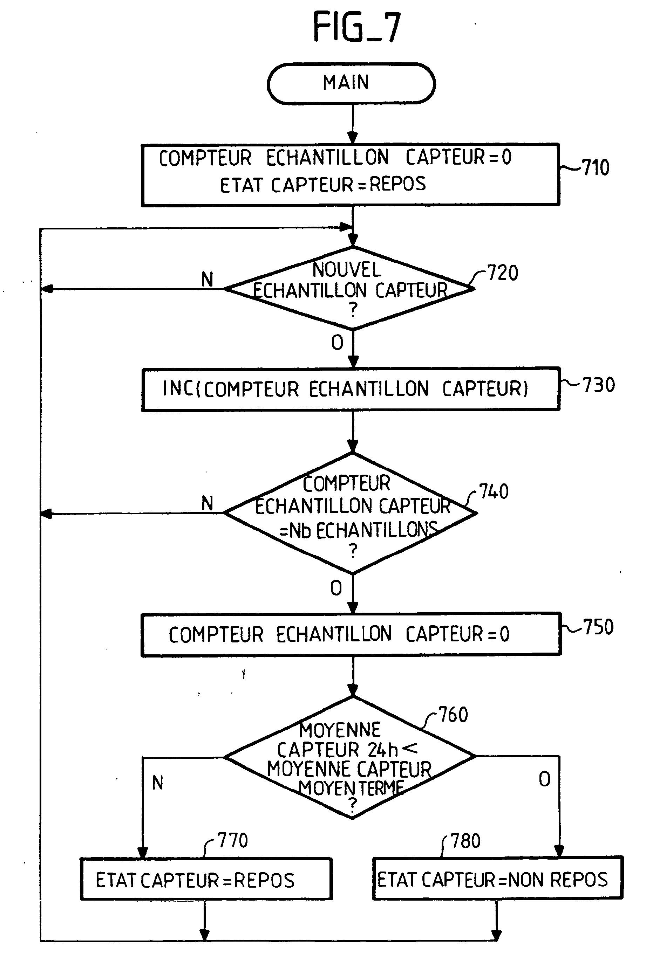

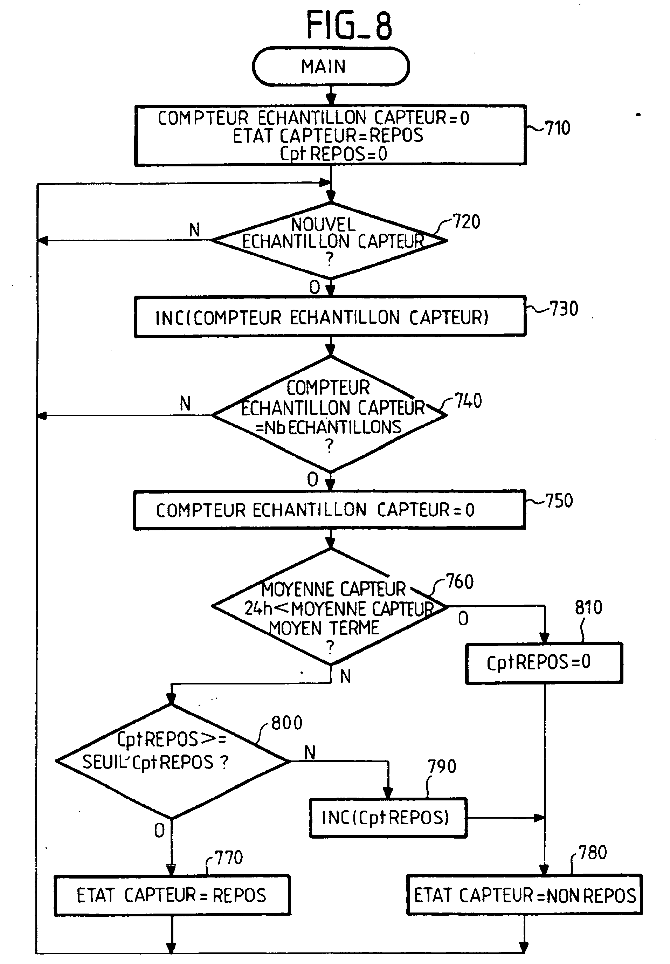

Le "critère d'activité capteur" défini plus haut, correspondant à une variable ÉtatCapteur, est déterminé conformément à l'organigramme de la figure 7 ou de la figure 8, selon le type de capteur servant à l'asservissement.

L'organigramme de la figure 7 correspond à l'asservissement par un capteur physiologique, comme dans l'exemple décrit jusqu'ici de la ventilation-minute.

Après une phase d'initialisation (étape 710) et après un nombre de cycles correspondant à la valeur de CompteurÉchantillonCapteur, typiquement après 32 cycles (étapes 720 à 750), le dispositif compare les variables MoyenneCapteur24h et MoyenneCapteurMoyenTerme (étape 760).

Si MoyenneCapteurMoyenTerme est inférieure à MoyenneCapteur24h, le dispositif considère que le niveau d'activité à moyen terme est inférieur au niveau d'activité moyen sur une période de 24 heures, que le patient est donc dans un état de repos avéré (par exemple une phase de sommeil nocturne) et positionne ÉtatCapteur à "Repos" (étape 770).

Dans le cas contraire, il considère qu'il n'y a pas repos, que le patient est toujours actif, et positionne ÉtatCapteur à "NonRepos" (étape 780).

Pour un capteur non physiologique (par exemple un capteur d'accélération), l'organigramme de la figure 7 est légèrement modifié, de la manière illustrée figure 8.

Dans ce cas, on prévoit un compteur CptRepos, remis à zéro à l'étape initiale 710 et incrémenté (étape 790) à chaque fois que l'on détecte que le patient est dans un état de repos avéré. Si cette situation se répète un nombre prédéterminé de fois, désigné SeuilCptRepos et typiquement de l'ordre de 12 répétitions (étape 800), alors on positionne effectivement ÉtatCapteur à "Repos" (étape 770). Dans le cas contraire, on réinitialise CptRepos (étape 810) et on positionne ÉtatCapteur à "NonRepos" (étape 780). On notera incidemment que l'organigramme de la figure 7 correspond en fait à une version simplifiée de celui de la figure 8, avec SeuilCptRepos = 0.

En variante, on peut remplacer les incrémentations et le test du nombre d'occurrences par un test opéré sur une période fixe donnée définie par l'horloge interne du dispositif, par exemple une période de 10 minutes.

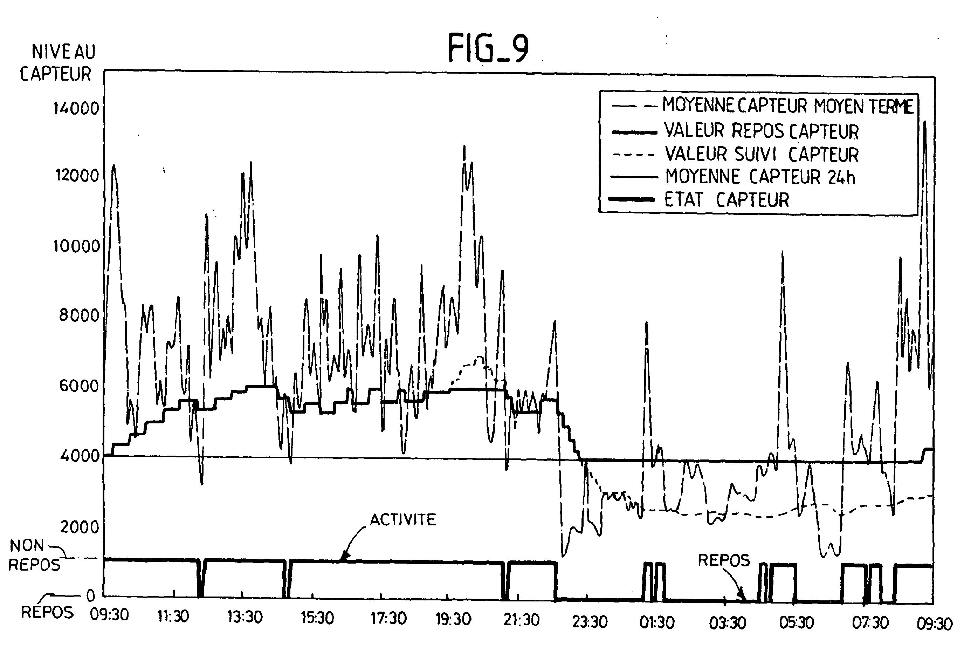

La figure 9 illustre un exemple d'évolution sur une période de 24 heures des différentes variables ValeurSuiviCapteur, MoyenneCapteurMoyenTerme, ValeurReposCapteur et MoyenneCapteur24h, ainsi que du critère ÉtatCapteur résultant déterminé conformément à l'invention.

On peut noter que, pendant la phase de sommeil entre 23 heures et 6 heures du matin, la variable ÉtatCapteur est de façon prépondérante à l'état "Repos".

L'information donnée par la variable ÉtatCapteur pourra être ainsi utilisée à l'intérieur du dispositif pour mettre en oeuvre diverses fonctions nécessitant ou exploitant la connaissance des phases de repos du porteur de l'appareil.

## Revendications

1. Dispositif médical implantable actif; comprenant :
- un capteur mesurant un paramètre servant au pilotage d'au moins une fonction dudit dispositif,
- des moyens d'acquisition d'échantillons successifs dudit paramètre mesuré par le capteur,
- des moyens de détermination d'un critère d'état du patient, lesdits moyens comprenant :
• des moyens de calcul, sur un premier intervalle de temps, d'une première valeur moyenne des échantillons acquis,
• des moyens de calcul, sur un second intervalle de temps supérieur au premier, d'une seconde valeur moyenne des échantillons acquis, et
• des moyens de comparaison de la première valeur moyenne et de la deuxième valeur moyenne,
dispositif **caractérisé en ce que** les moyens de détermination :
- comprennent un indicateur binaire, représentatif d'un état du patient propre à distinguer entre les phases de repos et les phases d'activité du patient et
- sont des moyens opérant ladite comparaison et positionnant ledit indicateur à partir de la comparaison, en donnant audit indicateur :
· une première valeur définissant un état de repos du patient si la première valeur moyenne est inférieure à la seconde valeur moyenne, et
· une seconde valeur définissant un état d'activité du patient dans le cas contraire.

2. Dispositif selon la revendication 1, dans lequel le premier et/ou le second intervalle de temps sont définis par une horloge interne du dispositif.

3. Dispositif selon la revendication 1, dans lequel le premier et/ou le second intervalle de temps sont définis par comptage d'un nombre d'échantillons acquis par le capteur.

4. Dispositif selon la revendication 3, dans lequel le premier intervalle de temps est défini par comptage d'un nombre d'échantillons compris entre 1 et 1024, de préférence 128 échantillons.

5. Dispositif selon la revendication 1, dans lequel le second intervalle de temps a une durée de l'ordre de 24 heures.

## Claims

1. An active implantable medical device, comprising:
- a sensor measuring a parameter for the control of at least one function of said device,
- means for the acquisition of successive samples of said parameter, which is measured by the sensor,
- means for determining a state criterion of the patient, said means comprising:
■ means for calculating, over a first time interval, a first average value of the acquired samples,
■ means for calculating, over a second time interval larger than the first, a second average value of the acquired samples, and
■ means for comparing the first average value and the second average value,
a device, **characterised in that** the determination means:
- comprises a binary indicator, representative for a state of the patient, for the distinction between the rest phases and the activity phases of the patient, and
- is a means performing said comparison and positioning said indicator on the basis of the comparison, by giving said indicator:
■ a first value defining a rest state of the patient if the first average value is lower than the second average value, and
■ a second value defining a state of activity of the patient in the opposite case.

2. The device of claim 1, wherein the first and/or the second time interval are defined by an internal clock of the device.

3. The device of claim 1, wherein the first and/or the second time interval are defined by counting a number of samples acquired by the sensor.

4. The device of claim 3, wherein the first time interval is defined by counting a number of samples between 1 and 1024, preferably 128 samples.

5. The device of claim 1, wherein the second time interval has a duration on the order of 24 hours.

## Patentansprüche

1. Aktive implantierbare medizinische Vorrichtung, mit:
- einem Sensor, der einen Parameter misst, welcher zur Steuerung wenigstens einer Funktion der Vorrichtung dient,
- Mitteln zur Erfassung aufeinander folgender Werte des durch den Sensor gemessenen Parameters,
- Mitteln zur Bestimmung eines Zustandskriteriums des Patienten, wobei die Mittel folgendes umfassen:
■ Mittel zur Berechnung eines ersten Mittelwertes der erfassten Werte über ein erstes Zeitintervall,
■ Mittel zur Berechnung eines zweiten Mittelwertes der erfassten Werte über ein zweites Zeitintervall, das größer ist als das erste, und
■ Mittel zum Vergleichen des ersten Mittelwertes mit dem zweiten Mittelwert,
Vorrichtung, die **dadurch gekennzeichnet ist, dass** die Bestimmungsmittel:
- einen für einen Zustand des Patienten repräsentativen binären Indikator zur Unterscheidung zwischen den Ruhephasen und den Aktivphasen des Patienten umfassen, und
- Mittel sind, die den Vergleich durchführen und den Indikator anhand des Vergleichs positionieren, indem sie dem Indikator:
■ einen ersten Wert geben, der einen Ruhezustand des Patienten definiert, wenn der erste Mittelwert geringer ist als der zweite Mittelwert, und,
■ im entgegen gesetzten Fall, einen zweiten Wert, der einen Aktivzustand des Patienten definiert.

2. Vorrichtung nach Anspruch 1, bei welcher das erste und/oder das zweite Zeitintervall durch eine interne Uhr der Vorrichtung definiert sind.

3. Vorrichtung nach Anspruch 1, bei welcher das erste und/oder das zweite Zeitintervall durch das Zählen einer Anzahl von durch den Sensor erfassten Werten definiert sind.

4. Vorrichtung nach Anspruch 3, bei welcher das erste Zeitintervall durch Zählen einer Anzahl Werte definiert ist, die zwischen 1 und 1024 liegt, vorzugsweise 128 Werte.

5. Vorrichtung nach Anspruch 1, bei welcher das zweite Zeitintervall eine Dauer von ungefähr 24 Stunden hat.
